# EUROPEAN PATENT SPECIFICATION

(11) **EP 1 063 969 B1**
(45) Date of publication and mention of the grant of the patent: **11.12.2002**
(21) Application number: 99913785.4
(22) Date of filing: 08.03.1999
(51) Int. Cl.: A61K 9/107, A61K 7/40, A61K 47/26

(54) **TOPICAL FORMULATION OF OIL-IN-WATER TYPE AS A CARRIER FOR PROVIDING A REDUCED IRRITANT EFFECT**
TOPISCHE FORMULIERUNGEN DES ÖL-IN-WASSER TYPS ALS TRÄGER ZUR VERMITTLUNG EINES REDUZIERTEN REIZEFFEKTS
FORMULATION DE PRODUIT EN APPLICATION LOCALE DU TYPE EMULSION HUILE DANS L'EAU

(30) Priority: 06.03.1998 SE 9800730
(43) Date of publication of application: 03.01.2001
(73) Proprietor: Lipocore Holding AB, 113 84 Stockholm (SE)
(72) Inventor: CARLSSON, Anders, S-112 38 Stockholm (SE); EK, Jenny, S-169 53 Solna (SE); HERSLÖF, Bengt, S-114 21 Stockholm (SE); KARLSSON, Ewa, S-194 60 Upplands Väsby (SE); NILSSON, Göran, S-112 52 Stockholm (SE)
(74) Representative: Larfeldt, Helene
(86) International application number: SE9900348
(87) International publication number: WO99044586

(56) References cited:
- EP-A1- 0 647 443
- WO-A1-95/20943

## Description

The present invention refers to a topical formulation of the oil-in-water emulsion type, containing a pharmaceutical or cosmetic compound which normally is strongly irritating to the skin, which after application on the skin surprisingly gives a reduced irritant effect of the incorporated compound.

### BACKGROUND OF THE INVENTION

There are a number of active substances which in spite of a pharmaceutically or cosmetologically advantageous effect after topical administration to some extent can be regarded as less acceptable owing to a local, irritating effect on the skin. Typical examples of such compounds are tretinoin and retinol, respectively.

Preferred formulations for topical administration of an active substance are creams and lotions, that is typically oil-in-water emulsions which spread readily on the skin, leave no detectable residue and adhere to the treated area without being tacky. Said emulsions normally consist of an oil phase, an aqueous phase and an emulsifier. ointments, which mainly comprises an oil phase, are greasy and form a greasy film on the skin preventing moisture loss. Gels which might be liposomal preparations do not contain any oil. Topical preparations of the oil-in-water emulsion type are generally more appreciated by the user from a cosmetic point of view, but have not been claimed to give any reduced irritant effect of incorporated substances of dermatological or cosmetological interest which are strongly irritant. From a dermatological standpoint oil-in-water emulsion type formulations are often preferred, particularly if the number of ingredients can be reduced to a minimum.

### PRIOR ART

The efficacy and local tolerability of liposomal tretinoin in man have been investigated, see Schäfer-Korting M., et al., Clin. Investig., Vol. 72, 1994, pp. 1086-1091. It was concluded that administration of the active substance liposomally encapsulated in a gel induced less skin irritancy than when tretinoin was administrated in a conventional gel formulation. The liposomal gel was well tolerated and was said to be an acceptable treatment for the patients.

EP-A-0 472 225, LVMH Recherche, discloses a pharmaceutical composition based on hydrated lipid lamellar phases or liposomes containing tretinoin or derivatives as the active material. The lamellar phases also contain sterols. The system is claimed to utilise the activity of the drug, while reducing the toxic and irritant effects.

US 5,545,407 discloses compositions for treating acne and other skin lesions. These compositions contain benzoyl peroxide, a compound for reducing the skin irritation associated therewith, and a topical carrier. The preferred class of compounds for reducing the skin irritation is tocopherol esters.

None of said references relate to emulsions of the oil-in-water type. Topical creams of the oil-in-water emulsion type have not previously been described as having the ability to reduce local skin irritation caused by an incorporated active substance. Moreover, there is a need of topical formulations, which are uncomplicated with respect to compositional design as well as manufacturing, such as oil-in-water emulsions, for reducing the irritant effect of the pharmacologically active substance. Furthermore, less complicated formulations have a major advantage in that they are less likely to cause irritant or hypersensitivity reactions and hence to be more acceptable as skin care preparations for therapeutic or cosmetic use.

WO 95/20943, Karlshamns LipidTeknik AB, discloses an oil-in-water emulsion comprising 0.01-50 % by weight of a galactolipid material as an emulsifier. Said emulsion is said to be useful as a carrier for active substances in a pharmaceutical composition but also in nutritional, cosmetic, food and agricultural products. The emulsions do not exhibit any unpleasant odour or taste and are stable towards oxidation. There is, however, nothing stated about the use of such emulsions in a topical cream and/or an ability to reduce skin irritation. EP-A-0 647 443 discloses an oil-in-water emulsion comprising an oily phase, an aqueous phase and an emulsifying system containing at least a first ester chosen among the glucose fatty acid esters and the alcylglucose fatty acid esters and at least a second ester of saccharose and fatty acids. Said emulsion is used for preparation of a cream in the treatment of skin diseases.

### DESCRIPTION OF THE INVENTION

The present invention refers to an oil-in-water emulsion for topical application to the skin comprising an emulsifier, an oil phase, and an aqueous phase, into which cosmetic or pharmaceutical substances can be incorporated for local treatment of various skin conditions and disorders.

It has surprisingly been found that a topical cream or lotion of the oil-in-water emulsion type, in which a galactolipid material is used as the emulsifier, and into which a pharmaceutical or cosmetic compound which normally is strongly irritating on the skin can be incorporated, after application on the skin gives a reduced irritant effect of the incorporated compound. Furthermore the stability, chemical as well as physical, of the topical formulations is very good.

The present invention refers to a topical formulation of the oil-in-water emulsion type, in which a variety of pharmaceutical or cosmetic compounds can be incorporated, comprising an oily material, an emulsifier and an aqueous phase, wherein the emulsifier is a glycolipid based material, and which after application on the skin gives a reduced irritant effect of the incorporated compound.

According to another aspect the invention refers to the use of a topical formulation of the oil-in-water type comprising an oily material, an aqueous phase and an emulsifier, wherein the emulsifier is a galactolipid material, as a carrier for providing a reduced irritant effect of an incorporated active substance on the skin.

Especially the invention refers to the use of a topical formulation, which can be a cream or a lotion, comprising 0.1-50 % by weight oily material, preferably 1-40 %, and 0.5-20 % by weight emulsifier.

No particular limitation is imposed on the oily material, that is the non-polar lipid material, of the formulation. Examples are vegetable oils, animal oils, fatty acids, synthetic oils, mineral oils, natural and synthetic glycerides, sterol esters, fatty alcohols, and other substances, including lipophilic drugs, obvious to a person skilled in the art, which can be emulsified using a polar lipid emulsifier.

Preferred oily materials to be emulsified are any fatty acid or a derivative thereof, such as vegetable oils of all types, such as oils from the seeds and beans of soybean, sunflower, rapeseed (canola), palm, corn, evening primrose, borage, groundnut, sesame, and similar.

There are also synthetic or semi-synthetic glycerides, propanediol derivatives, cholesteryl esters, other esters and other appropriate lipid materials. Another oily material for the emulsion is a medium-chain triacylglycerol (MCT) oil.

There are also many lipids such as free fatty acids, mono-, di- and triacylglycerols, phospholipids, cholesterol esters and lipids and oils of many other types which have therapeutic actions in themselves, such as tea tree oil, and which may be advantageously formulated in the form of a topical cream or optionally lotion. In this case the therapeutically active substance is the oily material, which can also have other bioactive properties.

The emulsifier according to the invention should be a glycolipid, preferably a galactolipid based material. Galactolipids can be defined as glycosylglycerides based on galactose and are well known constituents of plant cell membranes. The most important classes of these contain one to four sugars linked glycosidically to diacylglycerol. The two most abundant classes contain one and two galactose units, respectively, and are commonly known as mono- and digalactosyldiacylglycerol, MGDG and DGDG. Galactolipids, primarily DGDG and DGDG-rich materials, have been investigated and found to be a surface active material of interest in industrial application such as food, cosmetics, and pharmaceutical applications.

Synthetic diglycosyldiacylglycerols based on galactose, optionally in combination with other monosaccharide units, such as glucose, semi-synthetic, and natural glycosylglycerides, isolated from any source, can be used in accordance with the invention.

An intrinsic beneficial feature of the galactolipids is the galactose units comprising the polar head group in each lipid molecule, which may sterically stabilise the emulsion droplets in an emulsion. The galactose groups may also interact strongly with water and other polar substances, such as a water-soluble drug or a excipient, added to the emulsion.

WO 95/20943 describes the use of DGDG-rich material, a galactolipid material, as an emulsifier in oil-in-water emulsions. Said galactolipid material was prepared from cereals by extraction of the lipids with ethanol and a subsequent purification on a chromatographic column to pure DGDG or a DGDG-rich fraction of polar lipids. The galactolipid emulsifier consists of at least 50 % by weight digalactosyldiacylglycerols and a remainder of other polar lipids and can be used as the galactolipid emulsifier of the invention, preferably in an amount of 1.0-5.0 % by weight. The galactolipid material for instance consists of 70-80 % DGDG and 20-30 % other polar lipids.

According to a preferred embodiment of the invention the galactolipid emulsifier consists of 50-70 % by weight digalactosyldiacylglycerols and 30-50 % by weight other polar lipids. This material is manufactured by Scotia LipidTeknik AB, Stockholm, as CPL®-Galactolipid (registered trade mark owned by Scotia Holdings plc). A preferred topical formulation of the invention comprises CPL®-Galactolipid as the galactolipid material.

WO 97/11141 describes a method for producing a fractionated vegetable oil which is characterised in containing 10-90 % by weight of polar lipids, preferably 20-75 %, and a remainder of non-polar lipids. Said fractionated vegetable oil can also be used as the galactolipid emulsifier of the invention, preferably in an amount of 2.0-10 % by weight. The fractionated vegetable oil preferably contains more than 5 % by weight, preferably more than 20 %, glycolipids and preferably more than 3 % by weight, preferably more than 15 %, DGDG.

According to a preferred embodiment of the invention the galactolipid material consists of 40-60 % polar lipids and a remainder of non-polar lipids. A fractionated oat oil of this composition, consisting of a wide range of polar and amphiphilic lipids in a continuous triglyceride phase, is manufactured by Scotia LipidTeknik AB, Stockholm, as Galactolec™. A preferred topical formulation comprises Galactolec™ as the galactolipid material.

The galactolipid based emulsifier is a safe and non-toxic material for human and veterinary use. It is also an environmentally friendly material.

Topical formulations, such as creams and lotions, are prepared by using a polar lipid emulsifier either as the sole emulsifier or in combination with other amphiphilic compounds, that is co-surfactants. The formulation may also comprise optional additives known in the art for improving different aspects of the composition, such as thickening agents, preservatives, antioxidants, fragrance and the like.

The creams according to the invention are characterized by having excellent cosmetic properties. Furthermore they contain a minimum number of ingredients, without any stabilising ingredients known to give irritation or sensitisation of the skin. Despite the low number of ingredients the creams are extremely stable, with shelf lives of several years.

The active substance being irritating to the skin can be either water soluble or oil soluble or amphiphilic, and can be any type of pharmaceutical or cosmetological ingredient suitable for topical preparations, such as retinoids, e.g. tretinoin and retinol, vitamin D analogues, e.g. calcipotriol, benzoyl peroxide, dithranol, azelaic acid and clindamycin.

Topical creams according to the invention are prepared by conventional methods. For example, a 20 % (by weight) cream is prepared by adding the emulsifier to a triacylglycerol oil. The oil phase may also contain oil-soluble additives such as antioxidants and fragrance. The total emulsifier concentration is 1.5 % (by weight). The oil phase is then gently mixed. The continuous phase may be pure water or an aqueous solution containing water-soluble additives such as glycerol, preservatives and buffers. A water-soluble active compound, such as benzoyl peroxide, may then be added to the aqueous phase; consequently, an oil-soluble compound such as tretinoin is added to the oil phase. Alternatively, the drug may also be added to the final cream in an extemporaneous preparation. If necessary, the pH of the aqueous phase is adjusted. The oil phase as well as the aqueous phase are preheated to 70°C and then the oil phase is added to the aqueous phase under high-shear mixing. The pre-emulsion is then subjected to homogenisation at 200 psi. After cooling, the cream is transferred to suitable containers.

Formulations, that is creams and lotions, having the following, preferred compositions can be prepared accordingly.
Topical cream base giving an incorporated active substance a reduced skin irritant effect, comprising in % by weight

| | |
|---|---|
| Oily material | 10.0-30.0 % |
| Galactolipid emulsifier | 0.5-5 % |
| Thickener | 2.0-10.0 % |
| Preservative | 0.1-1.0 % |
| Water | ad 100 % |

Dermatological formulation having a reduced irritant effect, comprising in % by weight

| | |
|---|---|
| Tretinoin | 0.01-0.10 % |
| Oily material | 10.0-30.0 % |
| Galactolipid emulsifier | 0.5-5 % |
| Thickener | 2.0-10.0 % |
| Preservative | 0.1-1.0 % |
| Antioxidant | 0.02-0.3 % |
| Water | ad 100 % |

Different topical formulations with various non-polar oils as the cream base were formulated as described in Examples 1-2. Typical batch sizes are 0.5 to 1 kg. All concentrations are expressed in percent by weight.

### EXAMPLES OF FORMULATIONS

### Example 1

| Emulsifier: | | |
|---|---|---|
| CPL®-Galactolipid | 1.5 % | |

| Oil phase: | | |
|---|---|---|
| Tretinoin | 0.05 % | Active substance |
| CPL®-Evening Primrose oil | 20.0 % | Oily material |
| Cetostearyl alcohol | 7.0 % | Thickener |
| Glyceryl monostearate | 2.0 % | Thickener |
| Ascorbyl palmitate | 0.02 % | Antioxidant |

| Aqueous phase: | | |
|---|---|---|
| Glycerol | 2.0 % | Moisturiser |
| Methyl-p-hydroxybenzoate | 0.63 % | Preservative |
| Propyl-p-hydroxybenzoate | 0.07 % | Preservative |
| Water | ad 100 % | |

CPL®-Evening Primrose oil, CPL®-Galactolipid and ascorbyl palmitate were mixed in a beaker and stirred with a magnetic stirrer until the emulsifier had dispersed (30-60 min). The aqueous phase was prepared in another beaker and stirred with a magnetic stirrer. When the oil phase was clear cetostearyl alcohol and glyceryl monostearate were added. The oil phase and the aqueous phase were both heated to 70°C while stirring. Tretinoin was added to the oil phase when the oil phase had reached a temperature of 55°C. When the aqueous and oil phase both had reached 70°C, the aqueous phase was added to the oil phase during high-shear mixing (Polytron PT-MR 3000). After addition of the aqueous phase the emulsification (high-shear mixing) continued for 2 min at 15,000 rpm. The cream was allowed to cool in a water bath.

### Example 2

| Emulsifier: | | |
|---|---|---|
| Fractionated oat oil (Galactolec™) | 3.0 % | |

| Oil phase: | | |
|---|---|---|
| Tretinoin | 0.05 % | Active substance |
| CPL-Evening Primrose oil | 20.0 % | Oily material |
| Cetostearyl alcohol | 7.0 % | Thickener |
| Glyceryl monostearate | 2.0 % | Thickener |
| Ascorbyl palmitate | 0.02 % | Antioxidant |

| Aqueous phase: | | |
|---|---|---|
| Glycerol | 2.0 % | Moisturiser |
| Methyl-p-hydroxybenzoate | 0.63 % | Preservative |
| Propyl-p-hydroxybenzoate | 0.07 % | Preservative |
| Water | ad 100 % | |

The cream was prepared as described in Example 1.

The antioxidant used in Example 1 and 2 may very well be replaced by another suitable antioxidant, such as butylated hydroxytoluene (BHT), typically in an amount of 0.10-0.15 % by weight.

### Example 3

| Emulsifier: | | |
|---|---|---|
| CPL®-Galactolipid | 1.5 % | |

| Oil phase: | | |
|---|---|---|
| Tretinoin | 0.05 % | Active substance |
| Soybean oil | 20.0 % | Oily material |
| Cetostearyl alcohol | 7.0 % | Thickener |
| Glyceryl monostearate | 2.0 % | Thickener |
| Butylated hydroxytoluene | 0.15 % | Antioxidant |

| Aqueous phase: | | |
|---|---|---|
| Glycerol | 2.0 % | Moisturiser |
| Methyl-p-hydroxybenzoate | 0.63 % | Preservative |
| Propyl-p-bydroxybenzoate | 0.07 % | Preservative |
| Water | ad 100 % | |

In the oily phase paraffin liquid 20 %, Oenothera biennis (CPL-Evening primrose oil) 20 %, or a mixture of Oenothera biennis 10 % + soybean oil 10 %, can be substituted for the soybean oil, giving a tretinoin cream having equivalent properties.

Topical creams according to the invention containing tretinoin are very stable at 5°c and surprisingly stable at. room temperature (25°C).

### EXPERIMENTAL TEST

### Tests of skin irritation caused by tretinoin in two different cream formulations.

In order to evaluate the influence of the cream on skin irritation caused by tretinoin a Laser Doppler Flowmetry (LDF) technique was used (PeriFluxSystem 4000). By this technique the increase in cutaneous blood perfusion as a result of vasodilatation of the microvascular bed can be measured to assess and quantify the degree of skin irritation.

Ten healthy human volunteers participated in the test. To a small area (0.9 cm²) of the skin on the left cheek about 150 µg of the tretinoin cream in Example 1 was applied under occlusion. Similarly, 150 µg of a commercially available cream with 0.05% tretinoin (Aberela® cream from Janssen-cilag) was applied to the right cheek. Cutaneous blood perfusion was monitored prior to and during fifteen minutes following the topical administration of creams.

The increase in cutaneous blood perfusion during 15 minutes after application of two different tretinoin creams, 0.05 %, is stated in the table below.

**Table 1.**

| Increase in blood perfusion, in % | |
|---|---|
| Cream | Vascular response ± S.E. (95 % confidence level) |
| Aberela® | + 455 ± 119 |
| Cream (of Example 1) | + 49 ± 11 |

A pronounced increase of cutaneous blood perfusion was found after application of the Aberela® cream. The increase varied between the subjects from 2 to 10 times the baseline value and it reached a maximum level between 4 and 10 minutes following the application. During this period all subjects felt a more or less mild but distinct sense of burning or irritation. In some of the subjects a reddening of the exposed area of the cheek was observed which did not disappear until several hours after the removal of the patch.

Tretinoin in the formulation of the present invention (Example 1) caused only a very slight increase in cutaneous blood flow which on an average was not more than 1/10 of the increase found using the Aberela® cream. Although the increase in cutaneous blood perfusion varied between the ten individuals, all individuals showed a significantly higher increase using the Aberela® cream compared to the increase after having used the tretinoin formulation of the present invention. Furthermore the cream in Example 1 did not cause any sense of burning or irritation in the ten subjects.

The significantly lower degree of local irritation found after application of the tretinoin cream in Example 1 was verified in a further test on three healthy human volunteers. In this second test 55 mg of the two creams described above was gently massaged into the skin of the left and right cheek, the skin surface area of which was about 12 cm² on each side of the face. The creams were applied twice daily for two days. Cutaneous blood perfusion was monitored by LDF prior to the first application and on day three. This was immediately followed by a further application of the creams and the monitoring of cutaneous blood perfusion hourly during day three.

On day three and prior to the last application of the creams the cutaneous blood perfusion within the area where the Aberela® cream had been applied was found to be significantly elevated compared to the baseline value prior to the first application. No increase was found within the application area of the tretinoin cream in Example 1.

Following the last application of the creams on day three a similar result as in the first test was found. A pronounced increase of the cutaneous blood flow was found after application of the Aberela® cream, whereas application of the tretinoin cream in Example 1 caused only a slight increase in cutaneous blood flow.

Furthermore, from day three and the following couple of days the skin area onto which the Aberela® cream had been applied was in all three subjects characterised by being irritated. Redness, oedema, and scaling was found in all three subjects, although the extent of the reaction varied considerably between individuals. The skin area onto which the tretinoin cream in Example 1 had been applied showed no signs of irritation.

## Claims

1. Use of a formulation of the oil-in-water emulsion type comprising an oily material, an aqueous phase and a galactolipid material as an emulsifier, as a carrier for the preparation of a topical cream or lotion providing a reduced irritant effect of an incorporated pharmaceutically or cosmetically active substance on the skin.

2. Use according to claim 1, wherein the formulation comprises 0.1-50 % by weight of oily material and 0.5-20 % by weight of emulsifier.

3. Use according to claim 1 or 2, wherein the formulation comprises 1-40 % by weight of oily material and 0.5-10 % by weight of emulsifier.

4. Use according to any of claims 1-3, wherein the galactolipid material consists of at least 50 % by weight of digalactosyldiacylglycerols and a remainder of other polar lipids, and constitutes an amount of 1.0-5.0 % by weight of the formulation.

5. Use according to any of claims 1-4, wherein the galactolipid material consists of 50-70 % by weight digalactosyldiacylglycerols and 30-50 % other polar lipids.

6. Use according to any of claims 1-3, wherein the galactolipid material is a fractionated oat oil which consists of at least 15 % by weight of digalactosyldiacylglycerols and a remainder of other polar and non-polar lipids, and constitutes an amount of 2.0-10 % by weight of the formulation.

7. Use according to any of claims 1-3, and 6, wherein the galactolipid material is a fractionated oat oil which contains 40-60 % by weight polar lipids and a remainder of non-polar lipids.

8. Use according to any of claims 1-7, of a cream base, comprising in % by weight
| | |
|---|---|
| Oily material | 10.0-30.0 % |
| Galactolipid emulsifier | 0.5-5 % |
| Thickener | 2.0-10.0 % |
| Preservative | 0.1-1.0 % |
| Water | ad 100 % |

9. Use according to any of claims 1-8 for the preparation of a topical cream or lotion, incorporating a skin-irritating retinoid, especially tretinoin, as the active substance.

10. Use according to any of claims 1-8 for the preparation of a topical cream or lotion, incorporating benzoyl peroxide as the active substance.

## Patentansprüche

1. Verwendung einer Formulierung vom Öl-Wasser-Emulsionstyp, umfassend ein öliges Material, eine wäßrige Phase und ein Galactolipidmaterial als Emulgator, als Träger zur Herstellung einer topischen Creme oder Lotion, die eine reduzierte irritierende Wirkung einer inkorporierten pharmazeutischen oder kosmetisch wirksamen Substanz auf der Haut bereitstellt.

2. Verwendung gemäß Anspruch 1, wobei die Formulierung 0,1 bis 50 Gew.% eines öligen Materials und 0,5 bis 20 Gew.% Emulgator umfaßt.

3. Verwendung gemäß Anspruch 1 oder 2, wobei die Formulierung 1 bis 40 Gew.%.eines öligen Materials und 0,5 bis 10 Gew.% Emulgator umfaßt.

4. Verwendung gemäß einem der Ansprüche 1 bis 3, wobei das Galactolipidmaterial aus mindestens 50 Gew.% Digalactosyl-diacylglycerolen und einem Rest von anderen polaren Lipiden besteht und stellt eine Menge von 1,0 bis 5,0 Gew.% der Formulierung dar.

5. Verwendung gemäß einem der Ansprüche 1 bis 4, wobei das Galactolipidmaterial aus 50 bis 70 Gew.% Digalactosyl-diacylglycerolen und 30 bis 50 % anderen polaren Lipiden besteht.

6. Verwendung gemäß einem der Ansprüche 1 bis 3, wobei das Galactolipidmaterial ein fraktioniertes Haferöl ist, dieses besteht aus mindestens 15 Gew.% Digalactosyl-diacylglycerolen und einem Rest anderer polarer und nicht-polarer Lipide und stellt eine Menge von 2,0 bis 10 Gew.% der Formulierung dar.

7. Verwendung gemäß einem der Ansprüche 1 bis 3 und 6, wobei das Galactolipidmaterial ein fraktioniertes Haferöl ist, dieses enthält 40 bis 60 Gew.% polare Lipide und einen Rest von nicht-polaren Lipiden.

8. Verwendung gemäß einem der Ansprüche 1 bis 7, einer Cremebasis, umfassend in Gew.%:
| | |
|---|---|
| öliges Material | 10,0-30,0 % |
| Galactolipidemulgator | 0,5-5 % |
| Verdickungsmittel | 2,0-10,0 % |
| Konservierungsmittel | 0,1-1,0 % |
| Wasser | auf 100 % |

9. Verwendung gemäß einem der Ansprüche 1 bis 8 zur Herstellung einer topischen Creme oder Lotion, die inkorporiert ein hautirritierendes Retinoid, insbesondere Tretinoin, als aktive Substanz hat.

10. Verwendung gemäß einem der Ansprüche bis 8 zur Herstellung einer topischen Creme oder Lotion, die als wirksame Substanz Benzoylperoxid inkorporiert hat.

## Revendications

1. Utilisation d'une formulation de type émulsion huile dans l'eau comprenant un composé huileux, une phase aqueuse et un composé galactolipide en tant qu'émulsifiant, en tant qu'excipient pour la préparation d'une crème ou d'une lotion topique réduisant l'effet irritant d'une substance pharmaceutiquement ou cosmétiquement qui y est incorporée et qui est active sur la peau.

2. Utilisation selon la revendication 1, dans laquelle la formulation comprend de 0,1 à 50% en poids du composé huileux et de 0,5 à 20% en poids de l'émulsifiant.

3. Utilisation selon la revendication 1 ou 2, dans laquelle la formulation comprend de 1 à 40% en poids du composé huileux et de 0,5 à 10% en poids de l'émulsifiant.

4. Utilisation selon l'une quelconque des revendications 1 à 3, dans laquelle le composé galactolipide consiste en au moins 50% en poids de digalactosyldiacylglycérols et le reste d'autres lipides polaires, et représente une quantité de 1,0 à 5,0% en poids de la formulation.

5. Utilisation selon l'une quelconque des revendications 1 à 4, dans laquelle le composé galactolipide consiste en 50 à 70% en poids de digalactosyldiacylglycérols et 30 à 50% en poids d'autres lipides polaires.

6. Utilisation selon l'une quelconque des revendications 1 à 3, dans laquelle le composé galactolipide est une huile d'avoine fractionnée qui se compose d'au moins 15% en poids de digalactosyldiacylglycérols et le reste d'autres lipides polaires et non polaires, et représente une quantité de 2,0 à 10% en poids de la formulation.

7. Utilisation selon l'une quelconque des revendications 1 à 3, et 6, dans laquelle le composé galactolipide est une huile d'avoine fractionnée qui se compose de 40 à 60% en poids de lipides polaires et le reste de lipides non polaires.

8. Utilisation selon l'une quelconque des revendications 1 à 7, d'une crème comprenant en % en poids :
| | |
|---|---|
| Composé huileux | 10,0 à 30,0% |
| Emulsifiant galactolipide | 0,5 à 5% |
| Epaississant | 2,0 à 10,0% |
| Conservateur | 0,1 à 1,0% |
| Eau | jusqu'à 100% |

9. Utilisation selon l'une quelconque des revendications 1 à 8, pour la préparation d'une crème ou d'une lotion topique, incorporant un rétinoïde irritant pour la peau, en particulier la trétinoïne comme substance active.

10. Utilisation selon l'une quelconque des revendications 1 à 8, pour la préparation d'une crème ou d'une lotion topique, incorporant du peroxyde de benzoyle comme substance active.
